# EUROPÄISCHE PATENTSCHRIFT

(11) **EP 1 274 364 B1**
(45) Veröffentlichungstag und Bekanntmachung des Hinweises auf die Patenterteilung: **17.01.2007**
(21) Anmeldenummer: 01927885.2
(22) Anmeldetag: 06.04.2001
(51) Int. Cl.: A61C 8/00, A61F 2/30, A61B 17/68

(54) **MEDIZINISCHES IMPLANTAT**
MEDICAL IMPLANT
IMPLANT CHIRURGICAL

(30) Priorität: 19.04.2000 DE 10019338
(43) Veröffentlichungstag der Anmeldung: 15.01.2003
(73) Patentinhaber: Heraeus Kulzer GmbH, 63450 Hanau (DE)
(72) Erfinder: AUGTHUN, Michael, 45470 Mülheim (DE); PETERS, Manfred, 38302 Wolfenbüttel (DE); HASELHUHN, Klaus, 52064 Aachen (DE); SPIEKERMANN, Hubertus, 42718 Haan (DE)
(74) Vertreter: Kühn, Hans-Christian
(86) Internationale Anmeldenummer: PCT/EP2001/003948
(87) Internationale Veröffentlichungsnummer: WO 2001/080768

(56) Entgegenhaltungen:
- WO-A-00/64384
- DE-A- 3 300 764
- DE-A- 3 533 395
- DE-A- 3 611 139
- DE-A- 4 127 839
- DE-C- 3 241 963
- DE-U- 29 605 296
- US-A- 5 197 881
- US-A- 5 246 370

## Beschreibung

Die Erfindung betrifft ein Implantat zur Aufnahme eines Verbindungszapfens eines medizinischen Elements, mit einer Längsachse, einem distalen Ende und einem proximalen Ende, von dem aus sich eine Aufnahmeausnehmung für den Verbindungszapfen in das Innere des Implantats erstreckt, wobei das Implantat an seiner äusseren Mantelfläche kraft- oder formschlüssig mit der inneren Mantelfläche einer Aufnahmebohrung in einem Knochen eines menschlichen oder tierischen Körpers verbindbar ist, wobei der an die Aufnahmeausnehmung angepasste Verbindungszapfen in dieser durch Klemmung, Schrumpfung, Klebung oder Zementierung verankerbar ist, wobei das medizinische Element im verankerten Zustand mit einer Kontaktfläche eines in seinem Querschnitt über den Querschnitt der Aufnahmeausnehmung am proximalen Ende des Implantats radial nach aussen vorstehenden Kopfteils vollflächig an einer zugeordneten Kontaktffäche des Implantats an dessen proximalem Ende anliegt und die Aussenkontur des Querschnitts der Aufnahmeausnehmung zumindest in einem Abschnitt nicht kreisförmig ist.

Derartige Implantatsysteme sind beispielsweise im zahnmedizinischen Bereich weit verbreitet.

In diesem Fall wird das aufzunehmende medizinische Element z. B. von einem Ersatzzahn, einer Verschlusskappe oder einem Gingiva-Former gebildet. Das Implantat wird beispielsweise in den Kieferknochen eingeschraubt.

Ein Implantatsystem der eingangs beschriebenen Art ist beispielsweise aus der US 5,961,328 bekannt. Der Verbindungszapfen des medizinischen Elements und die Aufnahmeausnehmung des Implantats wirken nach einem Schlüssel-Schloss-Prinzip zusammen, wobei eine Verdrehsicherheit mit Hilfe von radial über den Verbindungszapfen vorstehenden Verschlusselementen erreicht wird, die im eingesetzten Zustand des medizinischen Elements in darin angepasste Vertiefungen im Kopfteil des Implantats eingreifen. Die Aufnahmeausnehmung weist ausgehend von dem proximalen Ende des Implantats zunächst einen konischen Abschnitt auf, an den sich ein zylindrischer Abschnitt anschliesst, dessen Querschnitt dem Querschnitt am Ende des konischen Abschnitts entspricht. Der in die Aufnahmeausnehmung einzusetzende Verbindungszapfen des medizinischen Elements weist eine sich im Querschnitt in Richtung auf sein distales Ende hin verjüngende Form auf, wobei die Aussenkontur des Verbindungszapfens in einem Längsschnitt konkav verläuft und eine sägezahnartige Oberflächenstruktur besitzt. Der Querschnitt des Verbindungszapfens ist jedoch nicht kreisförmig, sondern vielmehr fehlt im Vergleich zu einer rotationssymmetrischen Gestaltung einseitig ein Längsstreifen, so dass sich ein ebener Flächenabschnitt der Mantelfläche bildet, der parallel zur Längsachse des Verbindungszapfens verläuft.

Zum einen ist die Herstellung dieses bekannten Implantatsystems sehr aufwendig und daher teuer und zum anderen ist die Art der Krafteinleitung in den sich an das Kopfteil des medizinischen Elements anschliessenden konischen Abschnitt nicht optimal. Aufgrund der sich verjüngenden Querschnittsform ist nämlich die Möglichkeit, quer zur Längsachse des Verbindungszapfens gerichtete Kräfte, die insbesondere bei einer einseitigen Belastung des medizinischen Elements entstehen, aufzunehmen, eingeschränkt, wodurch es im ungünstigen Fall zu Kippbewegungen des medizinischen Elements kommen kann.

Der aus der WO99/29255 bekannte Ersatzzahn besteht aus einem Verbindungszapfen mit einem Unterteil, einem Zentralteil und einem Oberteil sowie, einer auf das Oberteil aufgeschobenen Krone. Der Ersatzzahn wird als Ganzes mit seinem konischen Unterteil in eine daran angepasste, ebenfalls konische Aufnahmebohrung in einem Implantat, das zuvor in den Kieferknochen eingesetzt und darin ausreichend eingeheilt sein muss, eingesetzt. Bei der Verbindung zwischen dem Verbindungszapfen und der Aufnahmebohrung kommt das Prinzip eines Klemmkonus zur Anwendung. Die Möglichkeit einer Verdrehung des Ersatzzahns um die Längsachse des Verbindungszapfens erlaubt es zwar, die Winkelstellung des Ersatzzahns in bezug auf eine Rotation um seine Hochachse beim Vorgang des Einsetzens exakt anzupassen, führt jedoch zu dem Nachteil, dass insbesondere bei einer grösseren Belastung eine hinreichende Verdrehsicherheit des Ersatzzahns in dem Implantat nicht gewährleistet sein kann.

Alternativ zu den vorgenannten Implantatsystemen ist eine Schraubverbindung zwischen dem Ersatzzahn und dem Implantat die am weitesten verbreitete Konnektierungsart, die sich durch ihre einfache Reversibilität auszeichnet. Als Nachteil ist jedoch hier der grosse Zeitaufwand für das Eindrehen der Befestigungsschrauben, insbesondere bei einer grösseren Anzahl von Ersatzzähnen sowie die oftmals unbefriedigende Dauerhaltbarkeit derartiger Systeme anzusehen. Fertigungsbedingt weisen nämlich nie sämtliche Gewindegänge der Schraubverbindung dasselbe Tragverhalten auf, weshalb es in einzelnen Abschnitten zu einer erhöhten und in anderen Abschnitten zu einer erniedrigten Kraft- bzw. Momentenübertragung kommt. Aus diesem Grunde entstehen undefinierte Belastungszustände, aus denen ungewollte Verformungen und Spannungsspitzen resultieren können. Entweder kann es hierdurch zur Beschädigung der Schraubverbindung oder aber zu einer Oberlastung der Verbindung zwischen Implantat und Knochen kommen, was schlimmstenfalls zu einem Totalverlust des Implantats führen kann.

DE4127839A1 betrifft eine Vorrichtung zum Befestigen von Zahnersatz an einem Implantat, wobei das Halteteil in das Implantat (1) in unterschiedlichen Drehwinkelstellungen formschlüssig einsetzbar ist. Das Halteteil kann insbesondere mit einem Mehrkantabschnitt in einer entsprechend ausgebildeten, abgesetzten Mehrkantaufnahme im Implantat in unterschiedlichen Drehwinkelstellungen einsetzbar sein, wobei ein im Anschluss an den Mehrkantabschnitt angeformtes Retentionsteil in eine Matrize des Implantates eingreift. Die Endbefestigung wird nicht näher erläutert.

US5246370 beschreibt ein Implantatsystem, bei dem das Abutment in eine trichterförmige Ausnehmung am Implantatpfosten paßt und dort festgeschraubt wir.

Der Erfindung liegt die Aufgabe zugrunde, ein Implantatsystem vorzuschlagen, bei dem sich die Verbindung zwischen dem medizinischen Element und dem Implantat auf einfache Weise herstellen lässt, wobei die Verbindung sich durch eine gleichmässige grossflächige Krafteinleitung sowie eine Verdrehsicherung auszeichnen soll.

Ausgehend von einem Implantat der eingangs beschriebenen Art wird diese Aufgabe erfindungsgemäss dadurch gelöst, dass die Aufnahmeausnehmung ausgehend von dem proximalen Ende des Implantats einen zylindrischen Abschnitt besitzt, an den sich ein sich im Querschnitt verjüngender Abschnitt anschließt 7 und dass der Verbindungszapfen ausgehend von dem Kopfteil des medizinischen Elements ebenfalls einen zylindrischen Abschnitt aufweist, an den sich ein sich im Querschnitt verjüngender Abschnitt anschliesst.

Die vollflächige Anlage im Bereich der Kontaktflachen bewirkt eine gleichmäßige Krafteinleitung von dem medizinischen Element in das Implantat, wodurch Spannungsspitzen und damit Materialüberlastungen und -schädigungen vermieden werden. Insbesondere bewirkt der zylindrische Abschnitt des Verbindungszapfens in Verbindung mit dem angepassten zylindrischen Abschnitt des Aufnahmeausnehmung eine sichere Fixierung, bei der auch eine extrem seitliche Krafteinleitung in das medizinische Element nicht zu Zugkraftkomponenten in der Trennfläche zwischen dem medizinischen Element und der inneren Mantelfläche des Implantats führen. Die Sicherheit gegen Lösen und Herausfallen ist bei dem erfindungsgemässen Implantat daher besonders gross, unabhängig davon, ob das medizinische Element eingeschrumpft, eingeklebt oder einzementiert wird.

Der nicht kreisförmige Querschnitt, der beispielsweise die Form eines (abgerundeten) Polygons (Dreieck, Viereck, Fünfeck usw.) oder einer Ellipse oder eines Ovals oder eines beliebigen anderen Flächengebildes haben kann, verhindert, dass eine Drehung des Verbindungszapfens des medizinischen Elements um seine Längsachse möglich ist. Durch die eindeutig definierte Winkelposition des medizinischen Elements in bezug auf eine Rotation um die Hochachse erübrigt sich ausserdem eine aufwendige Einstellung der Position des medizinischen Elements während des Einsetzens. Die eigentliche Verbindung zwischen Implantat und Verbindungszapfen erfolgt durch Klemmung, Schrumpfung, Verklebung oder Zementierung.

Gemäss einer Ausgestaltung der Erfindung wird vorgeschlagen, dass die in Richtung der Längsachse des Implantats gemessene Länge des zylindrischen Abschnitts des Verbindungszapfens geringfügig kleiner als die ebenfalls in Richtung der Längsachse des Implantats gemessene Länge des zylindrischen Abschnitts der Aufnahmeausnehmung ist. Hierdurch kann eine sichere Anlage der Kontaktflächen des medizinischen Elements einerseits und des Implantats andererseits erreicht werden, was für eine gleichmässige und grossflächige Krafteinfeitung wesentlich ist.

Die Differenz in den Längen sollte so bemessen sein, dass unter Berücksichtigung der Fertigungstoleranzen der einzelnen Elemente auch im ungünstigsten Fall stets eine Anlage im Bereich der Kontaktflächen eintritt. Eine zu grosse Wahl der Längsdifferenz sollte jedoch vermieden werden, um im Bereich der sich verjüngenden Querschnitte unnötig grosse Spalte zwischen dem Verbindungszapfen und der Aufnahmeausnehmung zu vermeiden.

Eine besonders vorteilhafte Ausgestaltung ist darin zu sehen, dass die Kontaktfläche kreisringförmig ist und in einer Ebene senkrecht zu der Längsachse des Implantats verläuft. Mit Hilfe einer solchen Ausgestaltung lässt sich der Aufwand bei der Herstellung des Implantats besonders gering halten. Um beim Einsetzen des Verbindungszapfens in die Aufnahmeausnehmung einen die Einschubbewegung unter Umständen behindernden Druckaufbau zu vermeiden, ist zwischen dem Verbindungszapfen in dessen verankertem Zustand und der Wandung der Aufnahmeausnehmung mindestens ein Entlüftungskanal gebildet, der sich von einer distalen Stirnseite des Verbindungszapfens bis zu dem proximalen Ende des Implantats erstreckt.

Besonders vorteilhaft ist es, wenn in der Kontaktfläche des Implantats mindestens eine Entlüftungsnut vorhanden ist, die sich von einem Entiüftungskanal bis zu der Mantelfläche des Implantats erstreckt. Auf diese Weise wird ein völlig ungehinderter Abfluss der von dem Verbindungszapfen verdrängten Luft gewährleistet.

Sinnvollerweise wird der Verbindungszapfen im Vergleich zur der Aufnahmebohrung so dimensioniert, dass der Verbindungszapfen auf jeden Fall so weit in die Aufnahmeausnehmung eingeführt werden kann, bis die Kontaktfläche seines Kopfteils an der Kontaktfläche des Implantats vollflächig zur Anlage kommt.

Eine Weiterbildung des erfindungsgemässen Implantats besteht darin, dass die Wandung der Aufnahmeausnehmung mit einer Mehrzahl von Ringnuten, die jeweils in Ebenen senkrecht zur Längsachse des Implantats verlaufen, oder mit einer wendelförmigen Nut versehen ist. Die Ringnuten bzw. die wendelförmige Nut sorgen zum einen dafür, dass der Montagevorgang, insbesondere in seiner letzten Phase, erleichtert wird. Insbesondere wird ein Ansprengen der zur Anlage aneinandergelangenden Flächen verhindert und eine bessere Führung des Verbindungszapfens erreicht. Ausserdem wird in den Ringnuten bzw. in der wendelförmigen Nut bei einer Fixierung des Verbindungszapfens mit Hilfe von Klebstoff, Klebstoffringen usw. ein wendelförmiger Klebstoffwulst erzeugt, über den Axialkräfte aufgenommen werden können.

Die Erfindung weiterausgestaltend, wird vorgeschlagen, dass die Wandung der Aufnahmeausnehmung mit mindestens einer Vertiefung versehen ist, mit der ein elastisches Clipelement eines medizinischen Elements, das heisst z. B. einer Verschlusskappe, eines Gingiva-Forrners, eines Abdruckpfostens und/oder eines provisorischen Ersatzzahns formschlüssig in Eingriff bringbar ist. Hierdurch können sämtliche Bauteile, die nur vorübergehend mit dem Implantat verbunden werden müssen, auf einfachste Weise in die Aufnahmeausnehmung eingesetzt und dadurch in einer genau vorbestimmten Position mit dem Implantat konnektiert werden. Mit dem Clipelement wird eine durch entsprechende Axialkraft wieder aufhebbare formschlüssige Verbindung geschaffen, weshalb die Clipmengen vorteilhafterweise leicht abgerundet sind, um ein zerstörungsfreies Trennen zu ermöglichen. Die Kraftübertragung erfolgt in der Übergangszeit über die Stirnflächen. Der Zeitaufwand für das Herstellen der Verbindung zwischen den nur vorübergehend verwendeten Bauteilen und dem Implantat wird drastisch reduziert, was sich insbesondere bei der gleichzeitigen Versorgung einer Mehrzahl von Implantaten in einem nicht unerheblichen Zeitgewinn äussert.

Beim erfindungsgemässsen Implantats besitzt der Querschnitt der Aufnahmeausnehmung in der Nähe des proximalen Endes des Implantats die Form eines abgerundeten Rechtecks und in der Nähe ihres Grundes die Form eines abgerundeten Quadrats, wobei der Übergang zwischen den vorgenannten Querschnittsformen ohne Sprünge erfolgt. Sinnvollerweise entspricht dabei die kürzere Kantenlänge des Rechtecks der Kantenlänge des Quadrats.

Nach der Erfindung wird des weiteren ein medizinisches Element vorgeschlagen, das mit einer Kontaktfläche eines in seinem Querschnitt über den Querschnitt der Aufnahmeausnehmung am proximalen Ende radial nach aussen vorstehenden Kopfteils vollflächig an einer zugeordneten Kontaktfläche des Implantats an dessen proximalen Ende anliegt und bei dem die Aussenkontur des Querschnitts des Verbindungszapfens zumindest in einem Abschnitt nicht kreisförmig ist.

Hierdurch wird auf einfachste Weise eine Verdrehsicherung erzielt und die Aufnahme auch grösserer Drehmomente ermöglicht, ohne dass die Verbindung gefährdet würde. Es besteht somit die Möglichkeit, das Implantat z. B. zusammen mit einer vormontierten Verschlusskappe oder einem Gingiva-Former in den Knochen einzusetzen.

Bei einer vorteilhaften Ausgestaltung besitzt der Verbindungszapfen in der Nähe seines proximalen Endes einen Querschnitt in Form eines Rechtecks, dessen Ecken stärker abgerundet oder gebrochen als die des Rechtecks des Querschnitts der Aufnahmeausnehmung an der im verankerten Zustand zugeordneten Stelle sind, und in der Nähe seines distalen Endes einen Querschnitt in Form eines abgerundeten Quadrats, dessen Ecken stärker abgerundet sind als die des Quadrats des Querschnitts der Aufnahmeausnehmung an der im verankerten Zustand zugeordneten Stelle.

Des weiteren wird vorgeschlagen, dass das distale Ende des Verbindungszapfens mindestens ein axial vorstehendes Clipelement aufweist, das im Einbauzustand mit einer Vertiefung in der Aufnahmeausnehmung des Implantats formschlüssig in Eingriff bringbar ist. Der Zeitaufwand bei der Montage fasst sich hierdurch gegenüber Schraubverbindungen bzw. provisorischen Klebe- bzw. Zementverbindungen erheblich reduzieren.

Die formschlüssige Verbindung über das Clipelement fasst sich auf besonders einfache Weise wieder aufheben, wenn ein im Einbauzustand ausserhalb der Aufnahmeausnehmung befindlicher Abschnitt in seiner Mantelfläche mit mindestens zwei gegenüberliegenden Vertiefungen versehen ist. In diese Vertiefungen lassen sich beispielsweise hakenförmige Kontaktelemente eines zangenförmigen Werkzeugs einbringen, mit dem sich z. B. eine eingeclipste Verschlusskappe, ein eingeclipster Gingiva-Former, Abdruckpfosten und/oder provisorischer Ersatzzahn auf einfache Weise durch Aufbringung einer entsprechenden Axialkraft wieder aus dem Implantat entfernen lassen.

Die Herstellung eines Formschlusses zwischen dem Werkzeug zum Entfernen der Verschlusskappe, des Gingiva-Formers, des Abdruckpfostens und/oder des provisorischen Ersatzzahns lässt sich vereinfachen, wenn die Vertiefungen eine Ringnut mit einem V-förmigen Querschnitt bilden.

Die Erfindung weiterausgestaltend, ist bei einem endgültigen Ersatzzahn vorgesehen, dass die Mantelfläche des Verbindungszapfens mit einer Mehrzahl von Ringnuten, die jeweils in einer Ebene senkrecht zur Längsachse des Verbindungszapfens verlaufen, versehen ist, die im verankerten Zustand des Ersatzzahns mit den Ringnuten in der Wandung der Aufnahmeausnehmung korrespondieren.

Die Erfindung wird nachfolgend anhand eines Ausführungsbeispiels, das in der Zeichnung dargestellt ist, näher erläutert.

Es zeigt :
- Fig. 1: ein Implantat mit einer eingeclipsten Verschlusskappe im Längsschnitt;
- Fig. 2: eine Draufsicht auf die Verschlusskappe;
- Fig. 3: eine Draufsicht auf das Implantat nach Entfernung der Verschlusskappe;
- Fig. 4: einen Querschnitt entlang der Linie IV-IV durch das Implantat gemäss Fig. 1;
- Fig. 5: einen Querschnitt entlang der Linie V-V durch das Implantat gemäss Fig. 1;
- Fig. 6: einen vergrösserten Ausschnitt des Eingriffsbereichs eines Clipelements;
- Fig. 7: einen vergrösserten und prinziphaft dargestellten Ausschnitt vom unterschiedlichen Beginn der Querschnittsverjüngung des Implantats und des Verbindungszapfens;
- Fig. 8: wie Fig. 1, jedoch mit aufgeclipstem Gingiva-Former;
- Fig. 9: wie Fig. 8, jedoch mit Schlitz im Gingiva-Former für transgingivale Einheilung;
- Fig. 10: einen Rohling für einen provisorischen Ersatzzahn in einem Laborimplantat;
- Fig. 11: wie Fig. 10, jedoch eines fertigen provisorischen Ersatzzahns;
- Fig. 12: wie Fig. 11, jedoch im erfindungsgemässen Implantat;
- Fig. 13: wie Fig. 10, jedoch eines endgültigen Ersatzzahns;

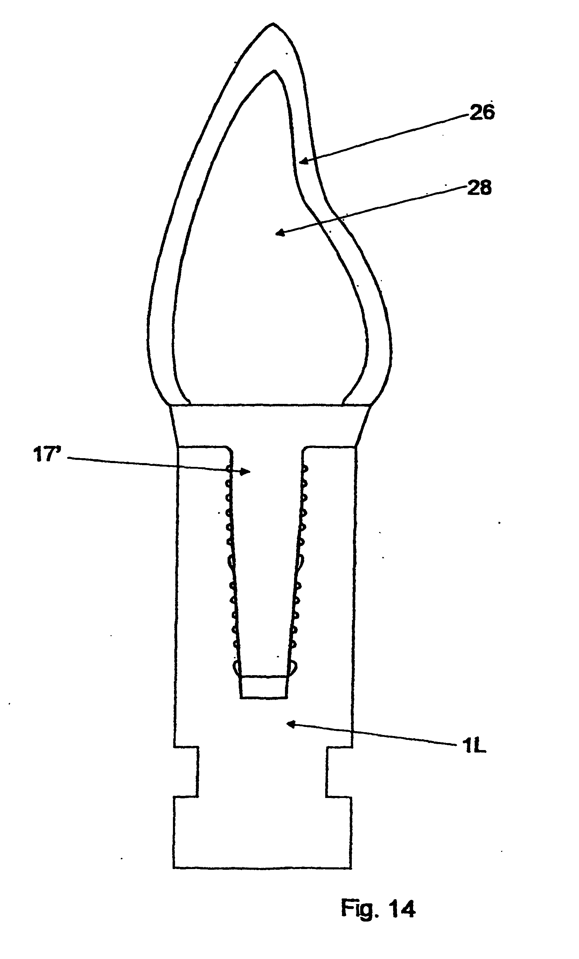

- Fig. 14: wie Fig. 11, jedoch eines endgültigen Ersatzzahns;
- Fig. 15: wie Fig. 12, jedoch eines endgültigen Ersatzzahns und
- Fig. 16: wie Fig. 15, jedoch mit einem Verbindungszapfen mit Ringnuten in der Mantelfläche.

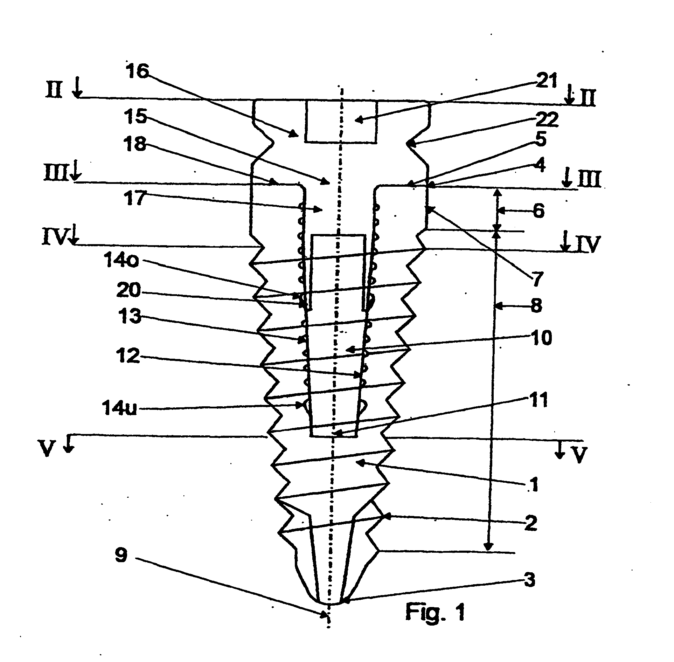

Den Fig. 1 bis 5 lässt sich ein aus Titan bestehendes Implantat 1 entnehmen, das eine ungefähr konische äussere Grundform besitzt und an seiner äusseren Mantelfläche mit einem Aussengewinde 2 versehen ist. Das Implantat 1 besitzt ein abgerundetes distales Ende 3 und ein proximales Ende 4, das von einer im wesentlichen kreisringförmigen Kontaktfläche 5 gebildet wird. In einem an die Kontaktfläche 5 anschliessenden Abschnitt 6 besitzt das Implantat aussen eine zylindrische Form mit einer hochglanzpolierten Mantelfläche 7. In einem darauf folgenden Gewindebereich 8 ist das Implantat 1 konisch ausgeformt. Ausgehend von der Kontaktfläche 5, erstreckt sich parallel zu einer Längsachse 9 des Implantats 1 eine Aufnahmeausnehmung 10, die über die gesamte Länge des Abschnitts 6 sowie einen Teil der Länge des Gewindeabschnitts 8 verläuft.

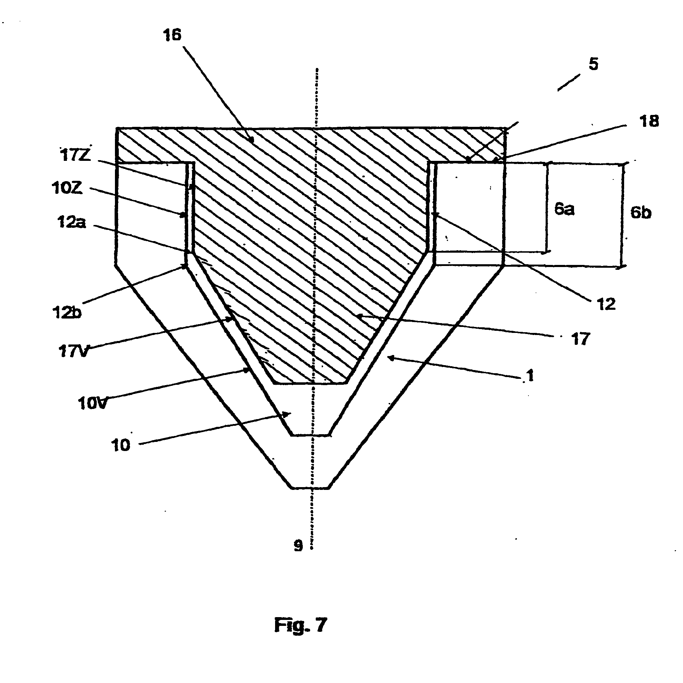

Aus der Prinzipdarstellung gemäss Figur 7 ergibt sich, dass die Aufnahmeausnehmung 10 ausgehend von dem proximalen Ende 4 des Implantats 1 zunächst einen zylindrischen Abschnitt 10Z besitzt, an den sich ein sich im Querschnitt verjüngender Abschnitt 10V anschliesst. Daran angepasst weist der Verbindungszapfen 17 eines einzusetzenden Ersatzzahns ausgehend von dem Kopfteil 16 ebenfalls zunächst einen zylindrischen Abschnitt 17Z auf, dessen Durchmesser geringfügig kleiner als der Durchmesser der Aufnahmeausnehmung 10 in deren zylindrischen Abschnitt 10Z ist. An den zylindrischen Abschnitt 17Z des Verbindungszapfens 17 schliesst sich ein sich im Querschnitt verjüngender Abschnitt 17V an. Da die Länge 6a des zylindrischen Abschnitts 17Z des Verbindungszapfens 17 geringfügig kleiner als die Länge 6b des zylindrischen Abschnitts 10Z der Aufnahmeausnehmung 10 bemessen ist, ist ausgeschlossen, dass es im Bereich der sich verjüngenden Abschnitte 10V, 17V zu einer Anlage des Verbindungszapfens 17 an die Wandung 12 der Aufnahmeausnehmung 10 kommt. Vielmehr ist durch diese Art der Komponentenpassung sichergestellt, dass stets eine vollflächige Anlage der Kontaktflächen 5 und 18 am proximalen Ende des Implantats 1 erzielt wird. Die Differenz zwischen den Längen 6b und 6a, d. h. der axiale Abstand zwischen den Umlaufkanten 12a an dem Verbindungszapfen 17 und 12b an der Aufnahmeausnehmung 10 des Implantats 1 wird so bemessen, dass auch im ungünstigsten Fall der Fertigungstoleranzen stets ein Minimalabstand in den Abschnitten 10V und 17V erhalten bleibt. Ansonsten ist die Längendifferenz möglichst gering zu halten, um das Spaltmass im Bereich der sich verjüngenden Abschnitte 10V und 17V gering zu halten.

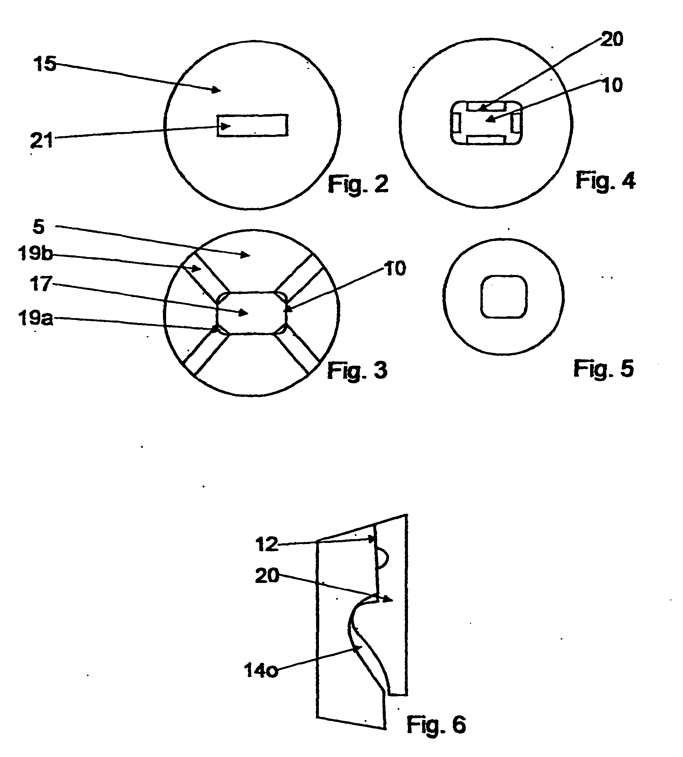

Wie sich den Fig. 3 und 7 entnehmen lässt, besitzt der Querschnitt der Aufnahmeausnehmung 10 im Bereich des zylindrischen Abschnitts 10Z durchgängig die Form eines abgerundeten Rechtecks. Beginnend mit dem Gewindebereich 8, verjüngt sich der Querschnitt der Aufnahmeausnehmung 10 in einem folgenden Abschnitt 10V dahingehend kontinuierlich, dass am Grund 11 der Aufnahmeausnehmung 10 der Querschnitt die Form eines abgerundeten Quadrats (vgl. Fig. 5) aufweist. Im Gewindebereich 8 erfolgt der Übergang von der abgerundeten rechteckigen zur abgerundeten quadratischen Querschnittsform kontinuierlich und ohne Sprünge.

Wie sich insbesondere aus Fig. 1 erkennen fasst, ist die Wandung 12 der Aufnahmeausnehmung 10 mit einer Vielzahl von Ringnuten 13 versehen, die senkrecht zur Längsachse 9 ausgerichtet sind. Des weiteren ist die Wandung 12 mit einer oberen und einer unteren Clipnut 14o und 14u versehen, deren Funktion später anhand der Fig. 6 erläutert wird.

In das in Fig. 1 dargestellte Implantat 1 ist eine Verschlusskappe 15 eingesetzt, die aus einem ungefähr zylinderförmigen Kopfteil 16 und einem koaxial hierzu ausgerichteten Verbindungszapfen 17 besteht, der sich in die Aufnahmeausnehmung 10 erstreckt. Eine Kontaktfläche 18 des Kopfteils 16 kommt an der Kontaktfläche 5 des Implantats 1kraftschlüssig zur Anlage.

Wie sich der Fig. 3 entnehmen lässt, weist der Verbindungszapfen 17 in einem oberen Abschnitt einen ungefähr rechteckförmigen Querschnitt auf, wobei die Eckbereiche derart gebrochen sind, dass in den Rundungsbereichen des Querschnitts der Aufnahmeausnehmung 10 zwischen dem Verbindungszapfen 17 und der Wandung 12 der Aufnahmeausnehmung 10 vier Entlüfungskanäle 19a gebildet werden. Beim Einschieben des Verbindungszapfens 17 in die Aufnahmeausnehmung 10 verdrängte Luft kann daher, ohne dass es zu einem den Montagevorgang behindernden Druckaufbau kommt, nach oben abgeführt werden, wobei die Luft durch vier radial nach aussen verlaufende Entlüftungsnuten 19b, die in die Stirnfläche 5 des Implantats eingebracht sind und mit den Entlüftungskanälen 19a kommunizieren, nach aussen entweichen kann.

Da die Verschlusskappe lediglich temporär nach der Implantation an dem Implantat 1 verbleibt, ist diese lediglich mit Hilfe von vier Clipelementen 20, die in die Clipnut140 eingreifen, mit dem Implantat 1 verbunden. Anstelle des in Fig. 1 gezeigten Eingriffs der Clipelemente 20 in die obere Clipnut 14o ist bei einem entsprechend verlängerten Verbindungszapfen 17 auch ein Eingriff in die untere Clipnut 14u möglich.

Die Verschlusskappe 15 wird bereits vom Hersteller des Implantats 1 in dieses eingesetzt und dient einerseits dazu, das Implantat 1 nach Anfertigung einer entsprechenden Bohrung im Knochen mit Hilfe eines Schraubendrehers, der in den in Fig. 2 gezeigten Schlitz 21 eingreift, einzudrehen. Aufgrund des annähernd rechteckförmigen Querschnitts des Verbindungszapfens 17 und der angepassten Aufnahmeausnehmung 10 ist eine Drehmomenteinleitung über die Verschlusskappe 15 in das Implantat 1 möglich. Nach der Implantation verbleibt die Verschlusskappe 15 am Implantat 1, um zum anderen die Aufnahmeausnehmung 10 vor äusseren Verschmutzungen zu schützen.

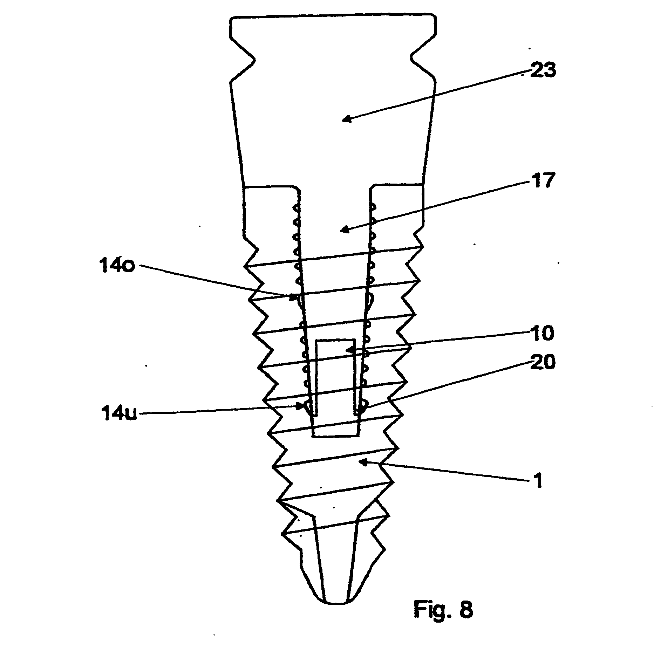

Circa drei bis sechs Monate nach Einsetzen des Implantats in den Kieferknochen ist die Einheilung so weit abgeschlossen, dass die die Verschlusskappe 15 abdeckende Schleimhaut in einer zweiten Operation wieder geöffnet werden kann. Die Verschlusskappe 15 wird entfernt, wozu mit Hilfe eines zangenartigen Werkzeugs in eine V-förmige Ringnut 22 in dem Kopfteil 16 eingegriffen wird und hierdurch die gesamte Verschlusskappe 15 durch einen leichten Ruck in axiale Richtung nach oben aus dem Implantat 1 entfernt wird. In die Aufnahmeausnehmung 10 des Implantats 1 wird nunmehr ein Verbindungszapfen 17 eines Gingiva-Formers 23 eingesetzt, wie er in Fig. 8 dargestellt ist. Das Befestigungsprinzip ist dasselbe wie bei der Verschlusskappe 15. Exemplarisch ist in Fig. 8 dargestellt, dass die Clipselemente 20 des Gingiva-Formers 23 in die untere Ringnut 14u einrasten. Ebenso ist jedoch ein Gingiva-Former 23 denkbar, bei dem die Clipelemente in die obere Ringnut 14o eingreifen.

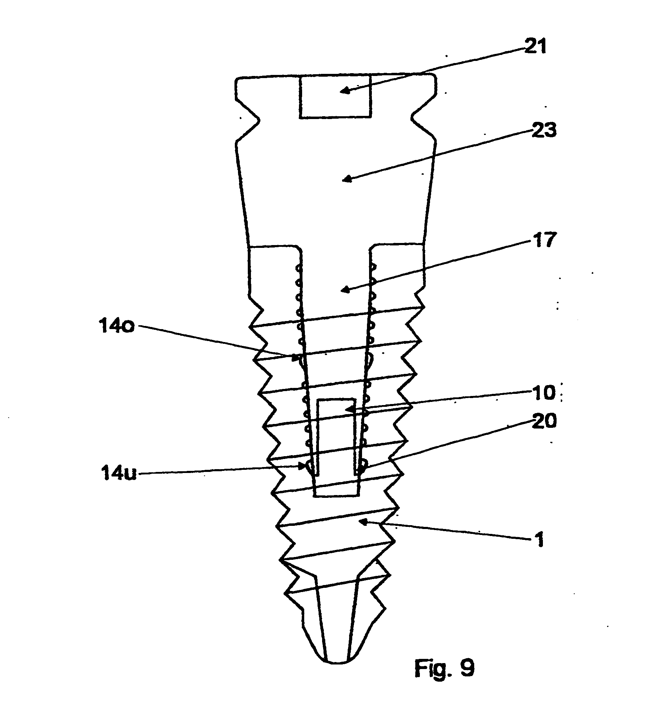

Fig. 9 zeigt einen Gingiva-Former mit Schlitz, der bei transgingivaler Einheilung des Implantats Verwendung findet.

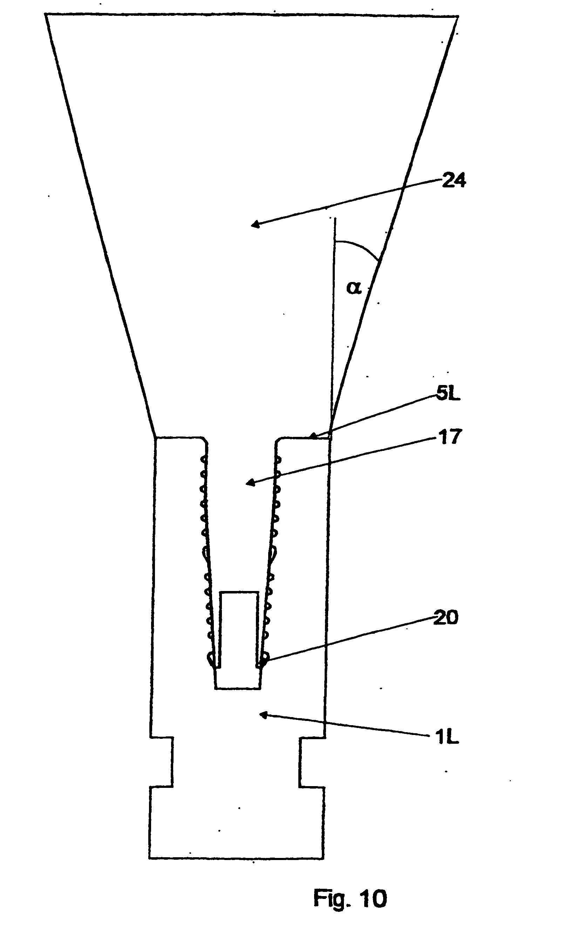

Fig. 10 zeigt ein Laborimplantat 1 L, in das ein Rohling 24 eines provisorischen Ersatzzahns ebenfalls mit Hilfe eines Verbindungszapfens 17 eingesetzt ist. Der Rohling 24 ist kegelstumpfförmig und erweitert sich, ausgehend von der Stirnfläche 5L des Laborimplantats 1 L, unter einem Winkel a von 15. Auf diese Weise lassen sich Schiefstellungen des Implantats 1 bzw. 1 L gegenüber den Nachbarzähnen in weiten Winkelbereichen in alle Richtungen ausgleichen. Der Verbindungszapfen 17 des Rohlings 24 ist ebenfalls mit Clipelementen 20 versehen, die ein unkompliziertes Fixieren und Entnehmen des Rohlings 24 gewährleisten.

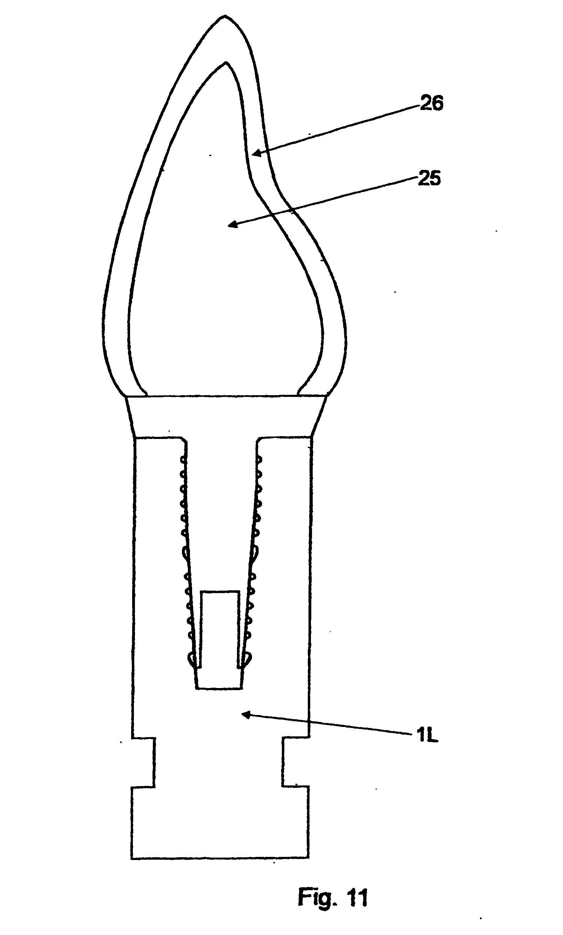

Fig. 11 zeigt einen fertigbearbeiteten provisorischen Ersatzzahn 25, der durch spanende Bearbeitung des Rohlings 24 in einem zahntechnischen Labor gefertigt wurde. Auf den geschliffenen Rohling ist eine äussere Keramikschicht 26 aufgebrannt.

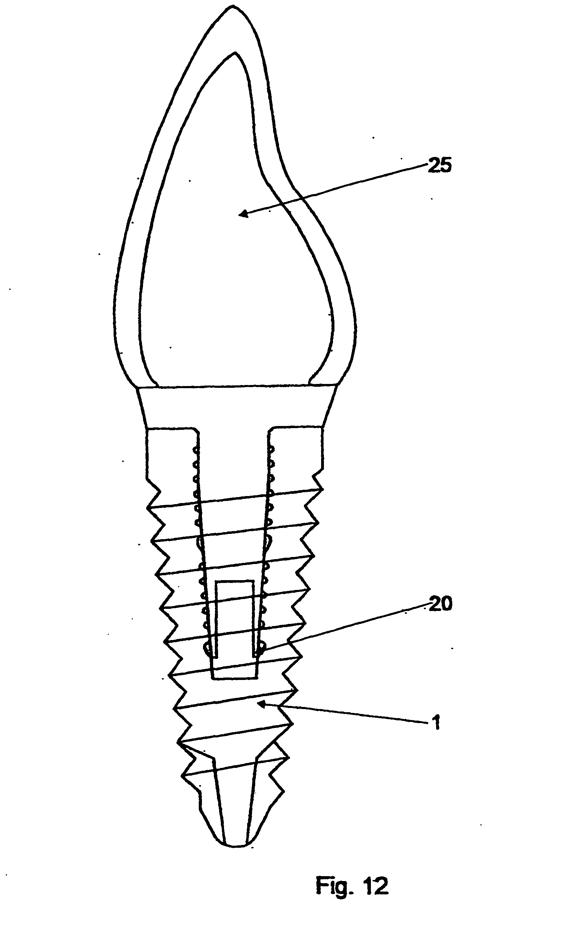

Der fertige provisorische Ersatzzahn 25 kann anschliessend beim Patienten in das Implantat 1 eingeführt und dort mit Hilfe der Clipelemente 20 so lange fixiert werden, bis der Patient mit dem endgültigen Ersatzzahn 28 versorgt werden kann (Fig. 12).

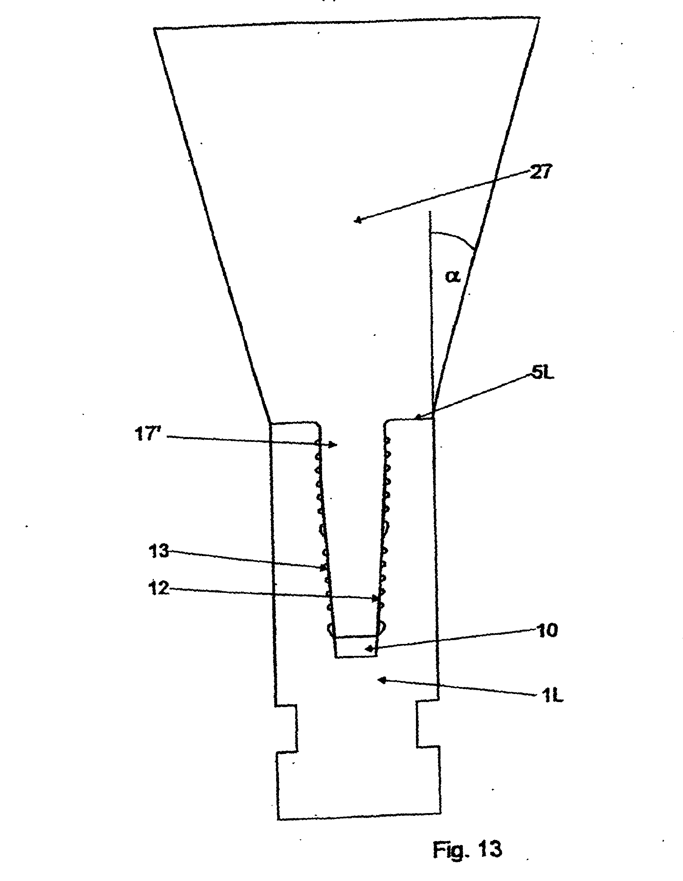

Dieser endgültige Ersatzzahn 28 wird wie der provisorische Ersatzzahn 25 aus einem in seinem Kopfteil kegelstumpfförmigen Rohling 27 (Fig. 13) hergestellt, der eine Schiefstellungskorrektur in alle Richtungen in weiten Winkelbereichen erlaubt. Der Verbindungszapfen 17' desendgültigen Ersatzzahns 28 bzw. dessen Rohlings 27 besitzt keine Clipelemente, sondern ist-abgesehen vom Bereich der Rundungen der Querschnittsform der Aufnahmeausnehmung 10 über die gesamte Länge des Verbindungszapfens 17' angepasst. Aufgrund der Ringnuten 13 in der Wandung 12 der Aufnahmeausnehmung 10 lässt sich der Verbindungszapfen 17 unproblematisch einführen, bis die Stirnfläche des Kopfteils des Rohlings 27 an der Stirnfläche 51-des Laborimplantas 1 L zur Anlage kommt. Unterschiedliche Anfänge der "Konizitäten" gewährleisten eine kraftschlüssige Verbindung über die Kontaktflächen 5 und 18 (s. auch Fig. 7). Ausgehend von dieser Einbauposition des Rohlings 27 im Laborimplantat 1 L kann die endgültige Form des Ersatzzahns 28, der wiederum eine aufgebrannte Keramikschicht 26 besitzt, hergestellt werden (Fig. 11).

Der Abschuss der Versorgung besteht darin, dass der endgültige Ersatzzahn 28 aus dem Laborimplantat 1 L entfernt und in das im Kieferknochen befindliche Implantat 1 eingesetzt wird.

Im Einbauzustand liegen die beiden Kontaktflächen 5 und 18 kraftschlüssig aneinander. Auch zwischen den äusseren Mantelflächen des Verbindungszapfens 17 und der Wandung 12 der Aufnahmeausnehmung 10 besteht ein Kraftschluss, der mit Hilfe von Klebstoff oder Zement hergestellt oder im Falle einer Schrumpfverbindung durch Klemmkräfte gebildet wird.

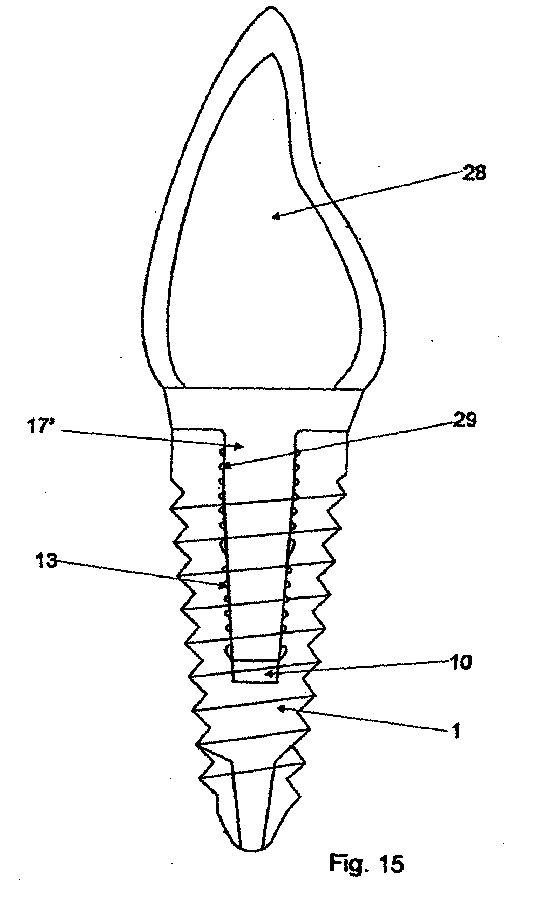

Gemäss Figur 15 werden die Mantelfläche 29 des Verbindungszapfens 17' und die Kontaktfläche 18 des Kopfteils mit einem geeigneten Klebstoff oder Zement bestrichen, der beim Einsetzen des Verbindungszapfens 17' die Ringnuten 13 in der Aufnahmeausnehmung 10 im wesentlichen vollständig ausfüllt. Nach Aushärtung des Klebstoffs existieren somit Klebstoffwulste, die auf dem Verbindungszapfen 17' anhaften und somit eine formschlüssige Verbindung mit dem Implantat 1 herstellen. Eine weitere kraftschlüssige Verbindung besteht über die Kontaktflächen 5 und 18, wobei der Klebstoff in diesem Bereich vorzugsweise nur so dünn aufgetragen wird, dass die Oberflächenrauhigkeiten der Kontaktflächen ausgefüllt werden und es trotz des Klebers zu einem Materialkontakt Implantat/Ersatzzahn kommt. Auf jeden Fall ist ein Hervorquellen von Klebstoff seitlich der Kontaktflächen 5 und 18 zu vermeiden.

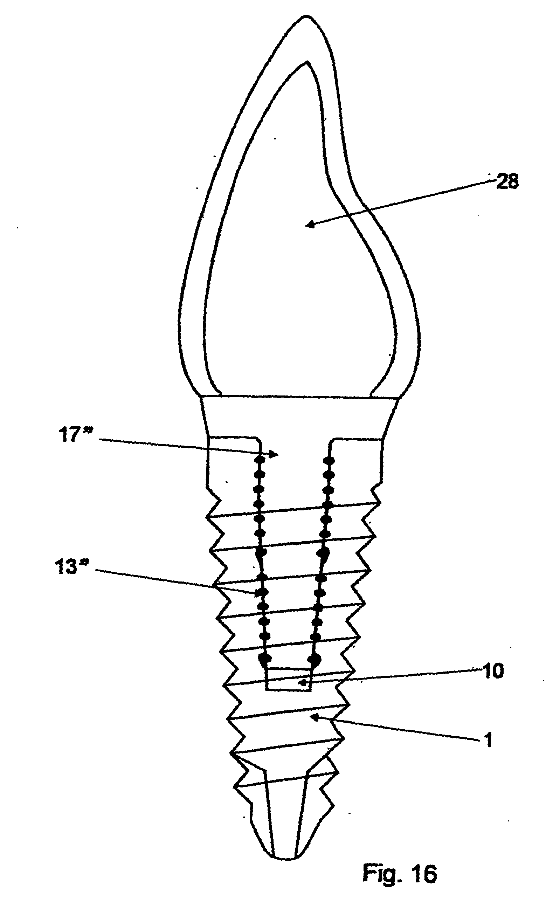

Die Festigkeit der Verbindung zwischen dem Ersatzzahn 28 und dem Implantat 1 kann weiter verbessert werden, wenn auch in der Mantelfläche des Verbindungszapfens 17" eine Mehrzahl von Ringnuten 13"vorhanden ist, die mit den Ringnuten 13 in der Wandung 12 der Aufnahmeausnehmung 10 korrespondieren. Eine derartige Ausgestaltung ist in Fig. 16 dargestellt. Es bilden sich hierbei im Querschnitt kreisförmige bzw. ovalförmige Klebstoffringe aus, die eine formschlüssige Verbindung zwischen dem Ersatzzahn 28 und dem Implantat 1 herstellen.

## Patentansprüche

1. Implantatsystem aufweisend
• ein Implantat und
• ein medizinisches Element
wobei das Implantat (1) zur Aufnahme eines Verbindungszapfens (17,17', 17") des medizinischen Elements vorgesehen ist,
wobei das Implantat aufweist:
eine Längsachse (9), ein distales Ende (3) und ein proximales Ende (4), von dem aus sich eine Aufnahmeausnehmung (10) für den Verbindungszapfen (17, 17', 17") in das Innere des Implantats (1) erstreckt,
wobei das Implantat (1) an seiner äusseren Mantelfläche kraft- oder formschlüssig mit der inneren Mantelfläche einer Aufnahmebohrung in einem Knochen eines menschlichen oder tierischen Körpers verbindbar ist,
wobei der an die Aufnahmeausnehmung (10) angepasste Verbindungszapfen (17,17', 17") in dieser durch Klemmung, Schrumpfung, Klebung oder Zementierung verankerbar ist, das medizinische Element im verankerten Zustand mit einer Kontaktfläche (18) eines in seinem Querschnitt über den Querschnitt der Aufnahmeausnehmung (10) am proximalen Ende (4) des Implantats (1) radial nach aussen vorstehenden Kopfteils (16) vollflächig an einer zugeordneten Kontaktfläche (5) des Implantats (1) an dessen proximalem Ende (4) anliegt und die Aussenkontur des Querschnitts der Aufnahmeausnehmung (10) zumindest in einem Abschnitt nicht kreisförmig ist,
wobei die Aufnahmeausnehmung (10) ausgehend von dem proximalen Ende (4) des Implantats (1) einen zylindrischen Abschnitt (10Z) besitzt, an den sich ein sich im Querschnitt verjüngender Abschnitt (10V) anschliesst, wobei der Verbindungszapfen (17,17',17") ausgehend von dem Kopfteil (16) des medizinischen Elements ebenfalls einen zylindrischen Abschnitt (17Z) aufweist, an den sich ein sich im Querschnitt verjüngender Abschnitt (17V) anschließt,
wobei der Querschnitt der Aufnahmeausnehmung (10) in der Nähe des proximalen Endes (4) des Implantats (1) die Form eines abgerundeten Rechtecks und in der Nähe ihres Grundes (11) die Form eines abgerundeten Quadrats besitzt, wobei der Übergang zwischen den vorgenannten Querschnittsformen ohne Sprünge erfolgt;
und wobei das medizinische Element mit einer Kontaktfläche (18) eines in seinem Querschnitt über den Querschnitt der Aufnahmeausnehmung (10) am proximalen Ende (4) des Implantats (1) radial nach aussen vorstehenden Kopfteils (16) vollflächig an einer zugeordneten Kontaktfläche (5) des Implantats (1) an dessen proximalen Ende (4) anliegt, die Aussenkontur des Querschnitts des Verbindungszapfens (17,17', 17") zumindest in einem Abschnitt, nicht kreisförmig ist und dass der Verbindungszapfen (17,17', 17") ausgehend von einem Kopfteil (16) einen zylindrischen Ab schnitt(17Z) aufweist, an den sich ein sich im Querschnitt verjüngender Abschnitt (17V) anschließt.

2. Implantatsystem nach Anspruch 1, **dadurch gekennzeichnet, dass** die in Richtung der Längsachse (9) des Implantats (1) gemessene Länge (6a) des zylindrischen Abschnitts (17Z) des Verbindungszapfens (17,17', 17") geringfügig kleiner als die ebenfalls in Richtung der Längsachse (9) des Implantats (1) gemessene Länge (6b) des zylindrischen Abschnitts (10Z) der Aufnahmeausnehmung (10) ist.

3. Implantatsystem nach Anspruch 1 oder 2, **dadurch gekennzeichnet, dass** die Kontaktfläche (5) kreisringförmig ist und in einer Ebene senkrecht zu der Längsachse (9) des Implantats (1) verläuft.

4. Implantatsystem nach einem der Ansprüche 1 bis 3, **dadurch gekennzeichnet, dass** zwischen dem Verbindungszapfen (17,17', 17") in dessen verankerten Zustand und der Wandung (12) der Aufnahmeausnehmung (10) mindestens ein Entlüftungskanal (19a) gebildet ist, der sich von einer distalen Stirnseite des Verbindungszapfens (17,17', 17") bis zu dem proximalen Ende (4) des Implantants (1) erstreckt.

5. Implantatsystem nach Anspruch 4, **dadurch gekennzeichnet, dass** in der Kontaktfläche (5) des Implantats (1) mindestens eine Entlüfungsnut (19b) vorhanden ist, die sich von einem Entlüftungskanal (19a) bis zu der Mantelfläche des Implantats (1) erstreckt.

6. Implantatsystem nach einem der Ansprüche 1 bis 5, **dadurch gekennzeichnet, dass** die Wandung (12) der Aufnahmeausnehmung (10) mit einer Mehrzahl von Ringnuten (13), die jeweils in Ebenen senkrecht zur Längsachse (9) des Implantats (1) verlaufen, oder mit einer wendelförmigen Nut versehen ist.

7. Implantatsystem nach einem der Ansprüche 1 bis 6, **dadurch gekennzeichnet, dass** die Wandung der Aufnahmeausnehmung (10) mit mindestens einer Vertiefung versehen ist, mit der ein elastisches Clipelement (20) eines medizinischen Elements formschlüssig in Eingriff bringbar ist.

8. Implantatsystem nach Anspruch 7, **dadurch gekennzeichnet, dass** die kürzere Kantenlänge des Rechtecks der Kantenlänge des Quadrats entspricht.

9. Implantatsystem nach einem der Ansprüche 1 bis 8, **dadurch gekennzeichnet, dass** es zur Aufnahme eines Ersatzzahns (25) vorgesehen ist.

10. Implantatsystem nach einem der Ansprüche 1 bis 9, wobei bei dem medizinischen Element der Verbindungszapfen (17,17', 17") in der Nähe seines proximalen Endes einen Querschnitt in Form eines Rechtecks besitzt, dessen Ecken stärker abgerundet oder gebrochen als die des Rechtecks des Querschnitts der Aufnahmeausnehmung (10) an der im Einbauzustand zugeordneten Stelle ist, und in der Nähe seines distalen Endes einen Querschnitt in Form eines Quadrats besitzt, dessen Ecken stärker abgerundet oder gebrochen sind als die des Quadrats des Querschnitts der Aufnahmeausnehmung an der im Einbauzustand zugeordneten Stelle.

11. Implantatsystem nach einem der Ansprüche 1 bis 10, wobei bei dem medizinischen Element das distale Ende des Verbindungszapfens (17) mindestens ein axial vorstehendes Clipelement (20) aufweist, das im Einbauzustand mit einer Vertiefung in der Aufnahmeausnehmung (10) eines Implantats (1) formschlüssig in Eingriff bringbar ist.

12. Implantatsystem nach einem der Ansprüche 1 bis 11, wobei bei dem medizinischen Element ein im Einbauzustand ausserhalb der Aufnahmeausnehmung befindlicher Abschnitt in seiner Mantelfläche mit mindestens zwei gegenüberliegenden Vertiefungen versehen ist.

13. Implantatsystem nach einem der Ansprüche 1 bis 12, wobei bei dem medizinischen Element die Vertiefungen eine Ringnut (22) mit einem V-förmigen Querschnitt bilden.

14. Implantatsystem nach einem der Ansprüche 1 bis 13, wobei das medizinische Element eine Verschlusskappe (15), ein Gingiva-Former (23), ein Abdruckpfosten und/oder ein Ersatzzahn (25) ist.

15. Implantatsystem nach Anspruch 14, wobei bei dem Ersatzzahn (28) die Mantelfläche des Verbindungszapfens (17"), mit einer Mehrzahl von Ringnuten (13") versehen ist, die jeweils in einer Ebene senkrecht zu der Längsachse des Verbindungszapfens (17") verlaufen und im verankerten Zustand des Ersatzzahns (28) mit den Ringnuten (13) in der Wandung (12) der Aufnahmeausnehmung (10) korrespondieren.

## Claims

1. Implant system comprising
• an implant
• a medical device
wherein the implant (1) is provided for receiving a connecting peg (17, 17', 17") of the medical device,
wherein the implant comprises:
a longitudinal axis (9), a distal end (3) and a proximal end (4), from which a receiving recess (10) for the connecting peg (17, 17', 17") extends into the interior of the implant (1), wherein the implant (1) can be connected non-positively or positively by its external surface to the internal surface of a receiving bore in a bone of a human or animal body, wherein the connecting peg (17, 17', 17") adapted to the receiving recess (10) can be anchored therein by clamping, shrinkage, adhesion or cementing, the anchored medical device rests with a contact face (18) of a top portion (16) protruding radially outwards in its cross-section beyond the cross-section of the receiving recess (10) at the proximal end (4) of the implant (1), over its entire area, on an associated contact face (5) of the implant (1) at the proximal end (4) thereof, and the external contour of the cross-section of the receiving recess (10) is not circular, at least in a portion,
wherein the receiving recess (10), extending from the proximal end (4) of the implant (1), has a cylindrical portion (10Z) which is followed by a portion (10V) that tapers in cross-section, and wherein, extending from the top portion (16) of the medical device, the connecting peg (17, 17', 17") also has a cylindrical portion (17Z) which is followed by a portion (17Z) that tapers in cross-section,
wherein the cross-section of the receiving recess (10), in the vicinity of the proximal end (4) of the implant (1), has the form of a rounded rectangle and, in the vicinity of its base (11), the form of a rounded square, the transition between the aforementioned cross-sectional shapes having no jumps;
and wherein the medical device rests with a contact face (18) of a top portion (16) protruding radially outwards in its cross-section beyond the cross-section of the receiving recess (10) at the proximal end (4) of the implant (1), over its entire area, on an associated contact face (5) of the implant (1) at the proximal end thereof, the external contour of the cross-section of the connecting peg (17, 17', 17") is not circular, at least in a portion, and in that, extending from a top portion (16), the connecting peg (17, 17', 17") has a cylindrical portion (17Z) which is followed by a portion (17V) that tapers in cross-section.

2. Implant system according to claim 1, **characterised in that**, measured in the direction of the longitudinal axis (9) of the implant (1), the length (6a) of the cylindrical portion (17Z) of the connecting peg (17, 17', 17") is slightly less than the length (6b) of the cylindrical portion (10Z) of the receiving recess (10), also measured in the direction of the longitudinal axis (9) of the implant (1).

3. Implant system according to either claim 1 or claim 2, **characterised in that** the contact face (5) is circular and extends in a plane perpendicular to the longitudinal axis (9) of the implant (1).

4. Implant system according to any one of claims 1 to 3, **characterised in that**, between the anchored connecting peg (17, 17', 17") and the wall (12) of the receiving recess (10), there is formed at least one ventilation duct (19a) which extends from a distal end face of the connecting peg (17, 17', 17") to the proximal end (4) of the implant (1).

5. Implant system according to claim 4, **characterised in that**, in the contact face (5) of the implant (1), there is provided at least one ventilation groove (19b) which extends from a ventilation duct (19a) to the surface of the implant (1).

6. Implant system according to any one of claims 1 to 5, **characterised in that** the wall (12) of the receiving recess (10) is provided with a plurality of annular grooves (13), which each extend in planes perpendicular to the longitudinal axis (9) of the implant (1), or with a helical groove.

7. Implant system according to any one of claims 1 to 6, **characterised in that** the wall of the receiving recess (10) is provided with at least one indentation, with which a resilient clip element (20) of a medical device can positively engage.

8. Implant system according to claim 7, **characterised in that** the shorter edge length of the rectangle corresponds to the edge length of the square.

9. Implant system according to any one of claims 1 to 8, **characterised in that** it is provided for receiving a replacement tooth (25).

10. Implant system according to any one of claims 1 to 9, wherein, in the case of the medical device, the connecting peg (17, 17', 17"), in the vicinity of its proximal end, has a cross-section in the form of a rectangle of which the corners are rounded or broken more markedly than those of the rectangle of the cross-section of the receiving recess (10) at the position allocated when fitted and, in the vicinity of its distal end, has a cross-section in the form of a square of which the corners are rounded or broken more markedly than those of the square of the cross-section of the receiving recess at the position allocated when fitted.

11. Implant system according to any one of claims 1 to 10, wherein, in the case of the medical device, the distal end of the connecting peg (17) has at least one axially protruding clip element (20) which, when fitted, can engage positively with an indentation in the receiving recess (10) of an implant (1).

12. Implant system according to any one of claims 1 to 11, wherein, in the case of the medical device, a portion which is located outside the receiving recess when fitted is provided with at least two opposing indentations in its surface.

13. Implant system according to any one of claims 1 to 12, wherein, in the case of the medical device, the indentations form an annular groove (22) having a V-shaped cross-section.

14. Implant system according to any one of claims 1 to 13, wherein the medical device is a cap 15, a gingiva shaper (23), an impression rod and/or a replacement tooth (25).

15. Implant system according to claim 14, wherein, in the case of the replacement tooth (28), the surface of the connecting peg (17") is provided with a plurality of annular grooves (13") which each extend in a plane perpendicular to the longitudinal axis of the connecting peg (17") and, when the replacement tooth (28) is anchored, correspond to the annular grooves (13) in the wall (12) of the receiving recess (10).

## Revendications

1. Système d'implant comprenant
■ un implant et
■ un élément médical,
dans lequel l'implant (1) est prévu pour recevoir un tenon de raccordement (17, 17', 17") de l'élément médical,
dans lequel l'implant présente
un axe longitudinal (9), une extrémité distale (3) et une extrémité proximale (4) à partir de laquelle s'étend, à l'intérieur de l'implant (1), un évidement de réception (10) pour le tenon de raccordement (17, 17', 17"),
dans lequel l'implant (1) peut être relié, au niveau de sa surface périphérique extérieure, par adhérence ou complémentarité de forme, avec la surface périphérique intérieure d'un alésage de réception dans un os d'un corps humain ou animal,
dans lequel le tenon de raccordement (17, 17', 17") adapté à l'évidement de réception (10) peut être ancré dans celui-ci par coincement, frettage, collage ou cimentation, l'élément médical s'appuyant de toute sa surface, dans l'état ancré, avec une surface de contact (18) d'une partie de tête (16) qui fait saillie avec sa section transversale radialement vers l'extérieur, au-delà de la section transversale de l'évidement de réception (10) à l'extrémité proximale (4) de l'implant (1), sur une surface de contact (5) associée de l'implant (1) à son extrémité proximale (4), et le contour extérieur de la section transversale de l'évidement de réception (10) n'étant pas de forme circulaire au moins sur un tronçon,
dans lequel l'évidement de réception (10) comporte, à partir de l'extrémité proximale (4) de l'implant (1), un tronçon cylindrique (10Z) auquel se raccorde un tronçon (10V) qui se rétrécit en section transversale, et le tenon de raccordement (17, 17', 17") présente également, à partir de la partie de tête (16) de l'élément médical, un tronçon cylindrique (17Z) auquel se raccorde un tronçon (17V) qui se rétrécit en section transversale,
dans lequel la section transversale de l'évidement de réception (10) présente à proximité de l'extrémité proximale (4) de l'implant (1), la forme d'un rectangle arrondi, et à proximité de son fond (11), la forme d'un carré arrondi, la transition entre les formes de section transversale précitées s'effectuant sans gradins ou discontinuités,
et dans lequel l'élément médical s'appuie de toute sa surface, avec une surface de contact (18) d'une partie de tête (16) qui fait saillie avec sa section transversale radialement vers l'extérieur, au-delà de la section transversale de l'évidement de réception (10) à l'extrémité proximale (4) de l'implant (1), sur une surface de contact (5) associée de l'implant (1) à son extrémité proximale (4), le contour extérieur de la section transversale du tenon de raccordement (17, 17', 17") n'est pas de forme circulaire au moins sur un tronçon, et le tenon de raccordement (17, 17', 17") présente à partir d'une partie de tête (16), un tronçon cylindrique (17Z) auquel se raccorde un tronçon (17V) qui se rétrécit en section transversale.

2. Système d'implant selon la revendication 1, **caractérisé en ce que** la longueur (6a) du tronçon cylindrique (17Z) du tenon de raccordement (17, 17', 17"), mesurée dans la direction de l'axe longitudinal (9) de l'implant (1), est très légèrement inférieure à la longueur (6b) du tronçon cylindrique (10Z) de l'évidement de réception (10), également mesurée dans la direction de l'axe longitudinal (9) de l'implant (1).

3. Système d'implant selon la revendication 1 ou 2, **caractérisé en ce que** la surface de contact (5) est de forme annulaire circulaire et s'étend dans un plan perpendiculaire à l'axe longitudinal (9) de l'implant (1).

4. Système d'implant selon l'une des revendications 1 à 3, **caractérisé en ce qu'**entre le tenon de raccordement (17, 17', 17"), dans l'état ancré de celui-ci, et la paroi (12) de l'évidement -de réception (10), est formé au moins un canal de purge d'air (19a), qui s'étend d'une face frontale distale du tenon de raccordement (17, 17', 17") jusqu'à l'extrémité proximale (4) de l'implant (1).

5. Système d'implant selon la revendication 4, **caractérisé en ce que** dans la surface de contact (5) de l'implant (1) existe au moins une rainure de purge d'air (19b), qui s'étend d'un canal de purge d'air (19a) jusqu'à la surface périphérique extérieure de l'implant (1).

6. Système d'implant selon l'une des revendications 1 à 5, **caractérisé en ce que** la paroi (12) de l'évidement de réception (10) est pourvue de plusieurs rainures annulaires (13), qui s'étendent respectivement dans des plans perpendiculaires à l'axe longitudinal (9) de l'implant (1), ou d'une rainure en forme d'hélice.

7. Système d'implant selon l'une des revendications 1 à 6, **caractérisé en ce que** la paroi de l'évidement de réception (10) est pourvue d'au moins un creux avec lequel peut être amené en prise par complémentarité de forme, un élément d'encliquetage (20) élastique d'un élément médical.

8. Système d'implant selon la revendication 7, **caractérisé en ce que** la longueur du côté le plus court du rectangle correspond à la longueur du côté du carré.

9. Système d'implant selon l'une des revendications 1 à 8, **caractérisé en ce qu'**il est prévu pour recevoir une prothèse de dent (25).

10. Système d'implant selon l'une des revendications 1 à 9, dans lequel sur l'élément médical, le tenon de raccordement (17, 17', 17") possède à proximité de son extrémité proximale, une section transversale sous la forme d'un rectangle dont les sommets sont arrondis ou chanfreinés plus fortement que ceux du rectangle de la section transversale de l'évidement de réception (10), au niveau de la zone associée dans l'état monté, et possède à proximité de son extrémité distale, une section transversale sous la forme d'un carré dont les sommets sont arrondis ou chanfreinés plus fortement que ceux du carré de la section transversale de l'évidement de réception, au niveau de la zone associée dans l'état monté.

11. Système d'implant selon l'une des revendications 1 à 10, dans lequel sur l'élément médical, l'extrémité distale du tenon de raccordement (17) présente au moins un élément d'encliquetage (20) en saillie axiale, qui dans l'état monté, peut être amené en prise par complémentarité de forme avec un creux dans l'évidement de réception (10) d'un implant (1).

12. Système d'implant selon l'une des revendications 1 à 11, dans lequel sur l'élément médical, un tronçon se trouvant, dans l'état monté, en-dehors de l'évidement de réception, présente dans sa surface périphérique, au moins deux creux opposés.

13. Système d'implant selon l'une des revendications 1 à 12, dans lequel sur l'élément médical, lesdits creux forment une rainure annulaire (22) d'une section transversale en forme de V.

14. Système d'implant selon l'une des revendications 1 à 13, dans lequel l'élément médical est un cabochon de fermeture (15), un élément de mise en forme ou de contention de gencive (23), un pivot de prise d'empreinte et/ou une prothèse de dent (25).

15. Système d'implant selon la revendication 14, dans lequel sur la prothèse de dent (28), la surface périphérique du tenon de raccordement (17'') est pourvue de plusieurs rainures annulaires (13"), qui s'étendent chacune dans un plan perpendiculaire à l'axe longitudinal du tenon de raccordement (17") et, dans l'état ancré de la prothèse de dent (28), sont en regard des rainures annulaires (13) dans la paroi (12) de l'évidement de réception (10).
